# EUROPEAN PATENT APPLICATION

(11) **EP 3 001 941 A1**
(43) Date of publication of application: **06.04.2016**
(21) Application number: 14801451.7
(22) Date of filing: 30.01.2014
(51) Int. Cl.: A61B 1/00, A61B 1/04, A61B 90/00

(54) **ENDOSCOPIC DEVICE AND ENDOSCOPIC DEVICE OPERATION METHOD**

(30) Priority: 23.05.2013 JP 2013108583
(71) Applicant: Olympus Corporation, Shibuya-ku Tokyo 151-0072 (JP)
(72) Inventor: TANAKA, Satoshi, Tokyo 151-0072 (JP)
(74) Representative: Gunzelmann, Rainer
(86) International application number: PCT/JP2014/052039
(87) International publication number: WO 2014/188740

(57) **Abstract**

An endoscope apparatus includes a distance information acquisition section (120), a degree-of-relation acquisition section (130), and a notification processing section (140). The distance information acquisition section (120) acquires distance information about the distance between a specific part and a treatment tool. The degree-of-relation acquisition section (130) acquires the degree of relation between the specific part and the treatment tool based on at least one of specific part information and treatment tool information, and the distance information, the specific part information being characteristic information about the specific part, and the treatment tool information being characteristic information about the treatment tool. The notification processing section (140) performs a notification process based on the degree of relation.

## Description

### TECHNICAL FIELD

The present invention relates to an endoscope apparatus, a method for operating an endoscope apparatus, and the like.

### BACKGROUND ART

An operation (surgery) support system has been known that notifies the user that a treatment tool has approached a safety-critical part (i.e., a part that may lead to serious consequences when damaged) during an operation (surgery). For example, Patent Document 1 discloses an operation support system that can notify the operator of the relative positional relationship between a treatment tool and a specific part (e.g., safety-critical part) for which contact with a treatment tool must be prevented during an operation. The operation support system disclosed in Patent Document 1 includes a model generation section that generates a tissue model from tissue image data acquired in advance, a distance calculation section that calculates the distance between the end of the treatment tool and the specific part, and a determination section that determines that the end of the treatment tool has approached the specific part when the distance is equal or less than a given threshold value, and notifies the operator that the treatment tool has approached the specific part based on position data that represents the positions of the tissue and the treatment tool during the operation.

### RELATED-ART DOCUMENT

### PATENT DOCUMENT

Patent Document 1: JP-A-2009-233240

### SUMMARY OF THE INVENTION

### TECHNICAL PROBLEM

However, since the above method notifies the operator that the treatment tool has approached the specific part (e.g., blood vessel) using only the distance between the treatment tool and the specific part, the notification process may be performed even when it is unnecessary to perform the notification process taking account of the characteristics of the treatment tool or the characteristics of the specific part, for example.

Several aspects of the invention may provide an endoscope apparatus, a method for operating an endoscope apparatus, and the like that can notify the user that a treatment tool has approached a specific part taking account of the characteristics of the treatment tool or the characteristics of the specific part.

### SOLUTION TO PROBLEM

According to one aspect of the invention, there is provided an endoscope apparatus comprising:
a distance information acquisition section that acquires distance information about a distance between a specific part and a treatment tool;
a degree-of-relation acquisition section that acquires a degree of relation between the specific part and the treatment tool based on at least one of specific part information and treatment tool information, and the distance information, the specific part information being characteristic information about the specific part, and the treatment tool information being characteristic information about the treatment tool; and
a notification processing section that performs a notification process based on the degree of relation.

According to one aspect of the invention, the degree of relation between the specific part and the treatment tool is acquired based on at least one of the specific part information (i.e., the characteristic information about the specific part) and the treatment tool information (i.e., the characteristic information about the treatment tool), and the distance information about the distance between the specific part and the treatment tool. This makes it possible to notify the user that the treatment tool has approached the specific part taking account of the characteristics of the treatment tool or the characteristics of the specific part.

According to another aspect of the invention, there is provided a method for operating an endoscope apparatus comprising:
acquiring distance information about a distance between a specific part and a treatment tool;
acquiring a degree of relation between the specific part and the treatment tool based on at least one of specific part information and treatment tool information, and the distance information, the specific part information being characteristic information about the specific part, and the treatment tool information being characteristic information about the treatment tool; and
performing a notification process based on the degree of relation.

### BRIEF DESCRIPTION OF DRAWINGS

FIG. 1 illustrates a basic configuration example of an endoscope apparatus.
FIG. 2 illustrates a configuration example of an endoscope apparatus (first embodiment).
FIG. 3 illustrates an example of a look-up table that links distance information and the degree of relation.
FIG. 4 illustrates an example of a degree-of-relation notification process.
FIG. 5 illustrates a configuration example of an endoscope apparatus (second embodiment).
FIG. 6 illustrates a configuration example of an endoscope apparatus (third embodiment).
FIG. 7 illustrates an example of a special light image.
FIG. 8 illustrates a configuration example of an endoscope apparatus (fourth embodiment).
FIG. 9 illustrates an example of a look-up table that links an incision capability, distance information, and the degree of relation.
FIG. 10 illustrates a configuration example of an endoscope apparatus (fifth embodiment).
FIG. 11 illustrates an example of a degree-of-relation notification process (sixth embodiment).

### DESCRIPTION OF EMBODIMENTS

Exemplary embodiments of the invention are described below. Note that the following exemplary embodiments do not in any way limit the scope of the invention laid out in the claims. Note also that all of the elements described in connection with the following exemplary embodiments should not necessarily be taken as essential elements of the invention.

### 1. Outline

An outline of the embodiments of the invention is described below. As described above, since the method disclosed in Patent Document 1 notifies the operator that the treatment tool has approached the specific part using only the distance between the treatment tool and the specific part, the notification process is performed even when it is unnecessary to perform the notification process taking account of the characteristics of the treatment tool or the characteristics of the specific part.

According to the method disclosed in Patent Document 1, it is necessary to acquire tissue image data in advance using MRI or the like, and a position detection device is required to detect treatment tool position data when performing an operation. Therefore, the method disclosed in Patent Document 1 has a problem in that a large-scale apparatus is required.

For example, a treatment tool may be detected from an image captured using a stereoscopic endoscope in order to implement a compact position detection device. However, when a procedure is performed using a knife or the like as the treatment tool, the end of the treatment tool may be hidden behind tissue, and may not be captured. Therefore, it may be difficult to detect that the treatment tool has approached the specific part by merely utilizing an image captured using a stereoscopic endoscope.

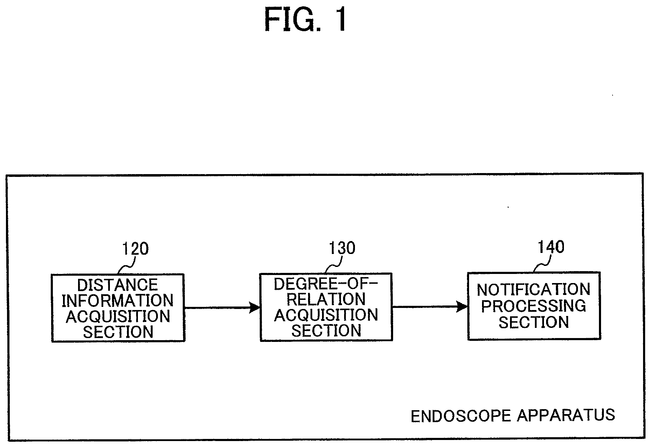

According to the embodiments of the invention, an endoscope apparatus includes a distance information acquisition section 120, a degree-of-relation acquisition section 130, and a notification processing section 140 (see FIG. 1). The distance information acquisition section 120 acquires distance information about the distance between a specific part and a treatment tool (e.g., knife). The degree-of-relation acquisition section 130 acquires the degree of relation between the specific part and the treatment tool based on at least one of specific part information and treatment tool information, and the distance information, the specific part information being characteristic information about the specific part, and the treatment tool information being characteristic information about the treatment tool. The notification processing section 140 performs a notification process based on the degree of relation.

This makes it possible to notify the user of the degree of relation between the specific part and the treatment tool based on the distance from the treatment tool to the specific part, the characteristics of the specific part, and the characteristics of the treatment tool. Therefore, it is possible to acquire the degree of relation taking account of the fact that the treatment tool has approached the specific part, the effect of the treatment tool on the specific part, and the necessity of notification, and notify the user of the degree of relation.

When the specific part and the treatment tool are detected from the captured image, the distance between the specific part and the treatment tool can be calculated from the captured image. Therefore, it is unnecessary to acquire an image of the specific part in advance using MRI or the like, and it is possible to implement a compact apparatus. Note that the position of the specific part and the position of the treatment tool need not necessarily be detected from the captured image. Various position detection methods may be applied to detect the position of the specific part and the position of the treatment tool.

It is possible to acquire the degree of relation between the treatment tool and the specific part even when the entire treatment tool is not captured within the captured image by utilizing the characteristic information about the treatment tool. For example, when physical size information about the treatment tool has been acquired as the characteristic information about the treatment tool, and part of the treatment tool is captured within the captured image, it is possible to estimate the position of the end of the treatment tool based on the size information, and estimate the distance from the end of the treatment tool to the specific part.

The term "specific part" used herein refers to a safety-critical part (or a part that requires special attention) that is included in an area that is treated using the treatment tool of the endoscope apparatus. Specifically, an injury to tissue may occur when the user has unintentionally damaged the specific part when performing a procedure (e.g., incision procedure or bleeding arrest procedure) using the treatment tool (e.g., electrosurgical knife or ultrasonic surgical knife). For example, the specific part may be a linear or tubular part such as a blood vessel, a nerve, or a urinary duct.

The distance information is information relating to the distance between the specific part and the treatment tool. The distance information need not necessarily be the distance from the treatment tool to the specific part. Specifically, the distance reference point need not necessarily be the end of the treatment tool, and may be a given position that is set arbitrarily. For example, the distance reference point may be the base of the treatment tool, the end of the imaging section (scope), or the like. Since the treatment tool is inserted into the end of the imaging section, and the shape and the size of the treatment tool are known in advance, the distance from the reference point can be used instead of the distance from the treatment tool. The end point of the distance is not limited to the position of the specific part that is closest to the treatment tool. For example, when a knife is used as the treatment tool, the end point of the distance may be the position of the specific part in the incision direction (along the extension of the tip of the knife). Since the effect of the treatment tool on the specific part (e.g., a possibility that bleeding occurs when the specific part is a blood vessel) is acquired as the degree of relation, the position of the specific part at which the specific part is affected by the treatment tool may be set to be the end point of the distance.

The distance information may be information about a two-dimensional distance, or may be information about a three-dimensional distance. The two-dimensional distance may be the distance between two points within an image, for example. The three-dimensional distance may be calculated by calculating the three-dimensional positions of two points by stereo imaging, and calculating the three-dimensional distance between the two points from the three-dimensional positions, for example. Alternatively, the distance in the depth direction may be estimated using an autofocus process, and the three-dimensional distance between two points may be calculated based on the distance in the depth direction and the distance between the two points within an image. When the specific part is a blood vessel, the depth from the surface of tissue to the blood vessel may be calculated using NBI (described later) or the like, and the three-dimensional distance may be estimated based on the depth and the distance between two points within an image. The distance may be calculated using the position of the specific part acquired in advance using MRI or the like, and the position of the treatment tool acquired using a position detection method (e.g., ultrasonic echo or GPS) instead of calculating the distance from an image captured in real time.

The term "degree of relation" used herein refers to an index that allows the user to determine the degree of importance of the effect of the treatment tool on the specific part. For example, when the specific part is a blood vessel, there is a possibility that the blood vessel is damaged by the treatment tool, and bleeding occurs. In this case, the degree of importance is high (i.e., the degree of relation is high) when the possibility that bleeding occurs is high, or when it is considered that a large amount of bleeding occurs. Specifically, since the possibility and the amount of bleeding change corresponding to the incision capability of the treatment tool and the attribute (e.g., thickness) of the blood vessel, the degree of relation is increased as the incision capability of the treatment tool increases, or the thickness of the blood vessel increases.

Note that the user (doctor) determines whether or not the effect of the treatment tool on the specific part is dangerous to the specific part, and the degree of relation is an index that assists the user in determining whether or not the effect of the treatment tool on the specific part is dangerous to the specific part. Specifically, when the user intentionally incises the specific part during a procedure, the user does not necessarily determine that the effect of the treatment tool on the specific part is dangerous to the specific part even when the degree of relation is high. On the other hand, when the user desires to perform a procedure without damaging the specific part, it is possible to prompt the user to carefully perform the procedure by increasing the degree of relation.

The term "characteristic information" used herein in connection with the treatment tool refers to information that corresponds to the characteristics of the treatment tool. For example, when the treatment tool is a knife, the characteristic information may be information that corresponds to the incision capability of the knife, such as the current that is caused to flow through the knife, the voltage applied to the knife, the material of the knife, the shape of the blade, the size of the blade, the procedure mode (e.g., bleeding arrest mode or incision mode) set to the knife, or the like. The characteristic information may be information about a part (e.g., lower gastrointestinal tract or upper gastrointestinal tract) for which the treatment tool is used, information that represents the type or the application of the treatment tool, ID information that is linked to the treatment tool, or the like.

The term "characteristic information" used herein in connection with the specific part refers to information that corresponds to the characteristics of the specific part that is included in the procedure target area. For example, when the specific part is a blood vessel, the characteristic information may be information that represents the type (e.g., artery, vein, or peripheral blood vessel) of the blood vessel, the thickness of the blood vessel, the part (e.g., lower gastrointestinal tract or upper gastrointestinal tract) where the blood vessel is present, the tissue (e.g., mucous membrane, fat, or muscle) where the blood vessel is present, or the like. For example, a blood vessel can be determined (recognized) from the captured image by image processing, and the thickness of the blood vessel can be determined by detecting the width (e.g., the number of pixels) of the blood vessel.

### 2. First embodiment

A detailed configuration according to a first embodiment of the invention is described below. An endoscope apparatus according to the first embodiment may be a gastroenterological endoscope apparatus that is inserted into a digestive organ (e.g., an upper gastrointestinal tract (e.g., esophagus or stomach) or a lower gastrointestinal tract (e.g., large intestine)), and used to perform a diagnosis/procedure, or may be a surgical endoscope apparatus that is inserted into an operative site (e.g., brain, abdominal part, or joint) during a surgical operation, and captures the operative site.

Although the first embodiment is described below taking an example in which the specific part is a blood vessel, the first embodiment is not limited thereto. Specifically, the specific part refers to a safety-critical part (or a part that requires special attention) that is included in an area that is treated using the treatment tool of the endoscope apparatus.

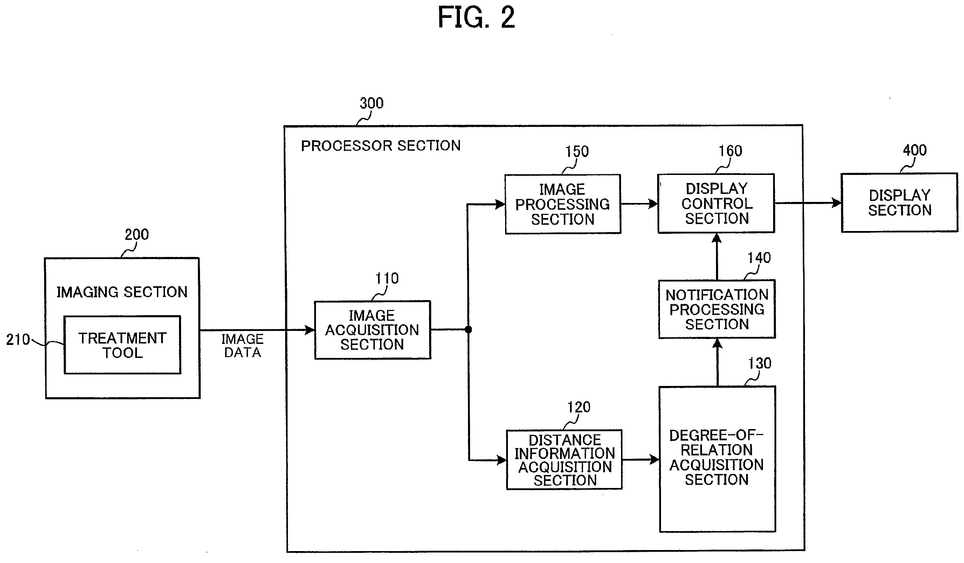

FIG. 2 illustrates a configuration example of the endoscope apparatus according to the first embodiment. The endoscope apparatus includes an imaging section 200 (scope section), a processor section 300, and a display section 400. In the first embodiment, a treatment tool and a blood vessel (specific part) are detected from the captured image, the two-dimensional distance between the treatment tool and the blood vessel in the image plane is calculated. The degree of relation is acquired based on the two-dimensional distance, and the user is notified of the degree of relation.

The imaging section 200 includes an image sensor (e.g., CCD or CMOS sensor). The imaging section 200 captures the object using the image sensor, and outputs the captured image data to the processor section 300. The imaging section 200 includes a treatment tool 210 (e.g., forceps or knife), an illumination optical system (e.g., light guide fiber and lens) (not illustrated in FIG. 2), and an operation section (e.g., an operation system for operating the end of the scope, an operation system for operating the treatment tool, and a mode setting button) (not illustrated in FIG. 2). The imaging section 200 is configured to be removable from the processor section 300. For example, the imaging section 200 can be exchanged corresponding to the target part and the like.

The processor section 300 performs image processing on the captured image, and controls each section of the endoscope apparatus. The processor section 300 includes an image acquisition section 110, a distance information acquisition section 120, a degree-of-relation acquisition section 130, a notification processing section 140, an image processing section 150, and a display control section 160.

The image acquisition section 110 receives the captured image (image data) output (transmitted) from the imaging section 200, and outputs the captured image to the image processing section 150 and the distance information acquisition section 120.

The image processing section 150 performs various types of image processing on the captured image. For example, the image processing section 150 performs a white balance process, a gamma correction process, an enhancement process, a scaling process, a distortion correction process, a noise reduction process, and the like.

The distance information acquisition section 120 detects a treatment tool and a blood vessel from the captured image, and calculates the two-dimensional distance between the detected treatment tool and the detected blood vessel. The treatment tool may be detected by utilizing the fact that a treatment tool is normally made of a metal. For example, the captured image is converted into a brightness (luminance) image, and pixels having a brightness value equal to or larger than a given threshold value are detected. The detected pixels are divided into a plurality of groups through a contour detection process, and a group having a pixel count equal to or larger than a given pixel count is detected as the treatment tool. A blood vessel may be detected based on the difference in brightness from an area in which a blood vessel is not present, for example. The end of the detected pixel group is determined to be the end of the treatment tool, and the distance from the end of the treatment tool to the blood vessel is calculated. Note that the treatment tool detection method and the blood vessel detection method are not limited thereto. For example, a treatment tool or a blood vessel may be detected from a color feature using a color difference image (Cr or Cb) or the like. When the specific part is a nerve, a nerve can be captured by administrating a medicine that causes a nerve to emit fluorescence, and detected from the captured image. When the specific part is a urinary duct, the structure of the urinary duct can be determined from an image by administering indocyanine green (ICG) (i.e., near infrared fluorescence probe), and observing fluorescence emitted from ICG that circulates inside the body, for example.

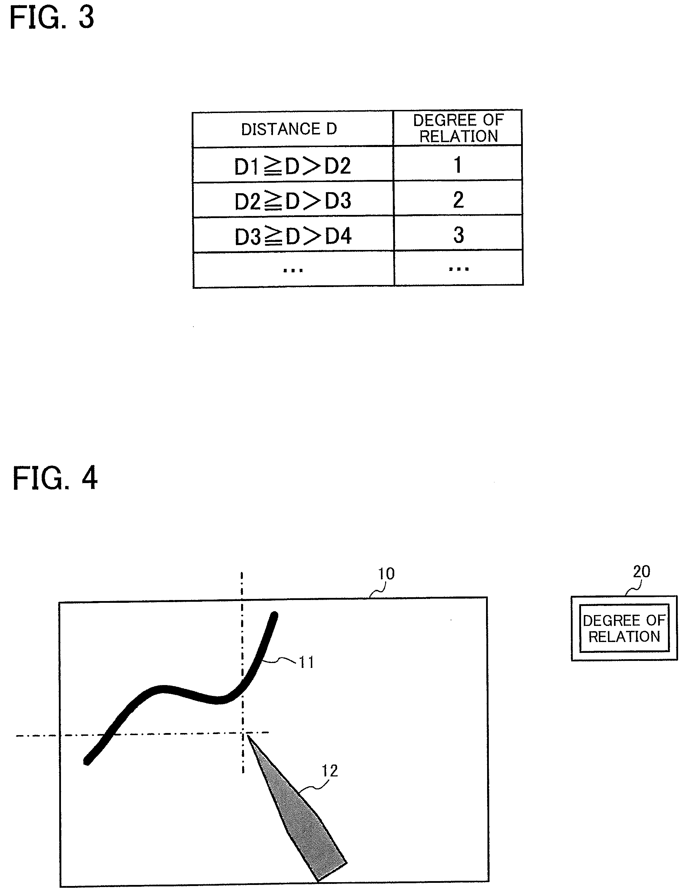

The degree-of-relation acquisition section 130 acquires the degree of relation based on the two-dimensional distance acquired by the distance information acquisition section 120. Specifically, the degree-of-relation acquisition section 130 increases the degree of relation as the two-dimensional distance from the treatment tool to the blood vessel decreases. For example, the degree-of-relation acquisition section 130 stores a look-up table (see FIG. 3), and converts the two-dimensional distance into the degree of relation by referring to the look-up table. Note that the relationship "D1>D2>D3>D4" is satisfied. The degree-of-relation acquisition section 130 may calculate the degree of relation using a function that links the two-dimensional distance to the degree of relation. For example, when the two-dimensional distance is referred to as D, the degree of relation is represented by f(D)=α/D. Note that α is a given coefficient.

The notification processing section 140 performs a process that notifies the user of the degree of relation acquired by the degree-of-relation acquisition section 130 through the display control section 160. FIG. 4 illustrates an example of the degree-of-relation notification process. An image displayed on the display section 400 includes an image display area 10 in which the captured image that includes a blood vessel 11, a treatment tool 12, and the like is displayed, and a degree-of-relation display area 20 in which the degree of relation is displayed. For example, the notification processing section 140 displays a red image (that represents that the degree of relation is high) in the degree-of-relation display area 20 when the degree of relation is equal to or higher than a first threshold value, displays a yellow image (that represents that the degree of relation is medium) in the degree-of-relation display area 20 when the degree of relation is lower than the first threshold value and equal to or higher than a second threshold value, and displays a black image (that represents that the degree of relation is low) in the degree-of-relation display area 20 when the degree of relation is lower than the second threshold value. Note that the degree-of-relation notification process need not necessarily be performed using an image. For example, the degree-of-relation notification process may be performed using sound, vibrations, or light emitted from an LED.

According to the first embodiment, the distance between the treatment tool and the specific part is derived as the degree of closeness by calculating the position information about the treatment tool and the specific part (e.g., blood vessel) from the image data output from the endoscopic scope, and acquiring the distance information about the treatment tool and the specific part. The user is notified of the degree of closeness as the degree of relation. The user can reduce the risk that the treatment tool approaches or comes in contact with the specific part by mistake, and damages the specific part (e.g., bleeding from a blood vessel) by operating the treatment tool while observing the degree of relation displayed on the monitor.

### 3. Second embodiment

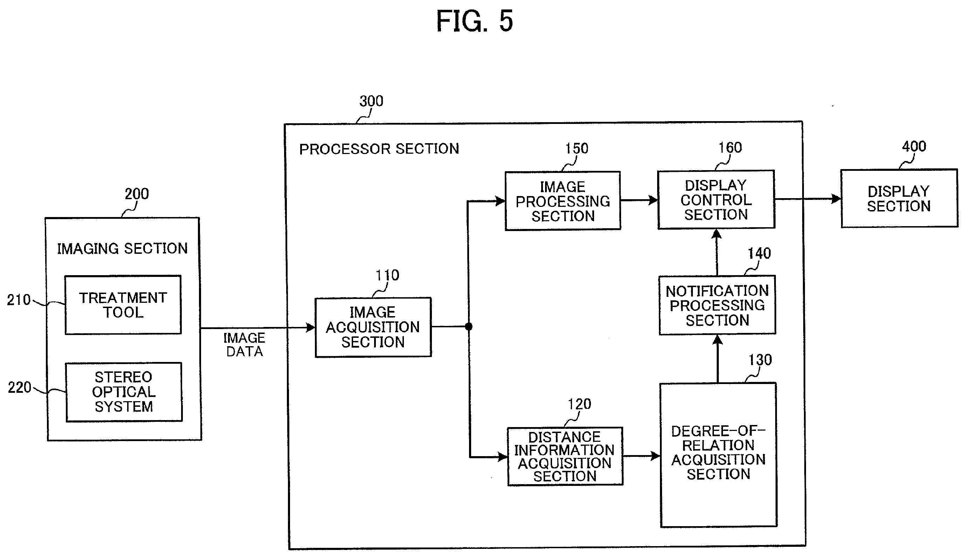

FIG. 5 illustrates a configuration example of an endoscope apparatus according to a second embodiment of the invention. The endoscope apparatus includes an imaging section 200, a processor section 300, and a display section 400. The processor section 300 includes an image acquisition section 110, a distance information acquisition section 120, a degree-of-relation acquisition section 130, a notification processing section 140, an image processing section 150, and a display control section 160. Note that the same elements as those described above in connection with the first embodiment are indicated by the same reference signs (symbols), and description thereof is appropriately omitted.

In the second embodiment, the three-dimensional distance (relative distance) between a treatment tool and a blood vessel is calculated, and the degree of relation is acquired based on the calculated three-dimensional distance.

Specifically, the imaging section 200 includes a treatment tool 210, and a stereo optical system that can capture a stereo image. The stereo optical system 220 includes two imaging optical systems that are disposed to have a parallax, for example. The distance information acquisition section 120 performs a matching process on a stereo image having a parallax to calculate the distance in the depth direction at each position within the image. The distance information acquisition section 120 detects a treatment tool and a blood vessel from the stereo image, detects the three-dimensional position of the treatment tool and the three-dimensional position of the blood vessel based on the distance in the depth direction that has been detected by the matching process, and calculates the three-dimensional distance between the treatment tool and the blood vessel. The degree-of-relation acquisition section 130 converts the three-dimensional distance into the degree of relation in the same manner as described above in connection with the first embodiment, and outputs the degree of relation to the notification processing section 140.

Note that the three-dimensional position of the treatment tool and the three-dimensional position of the blood vessel may be detected using a method other than the stereo imaging method. For example, the imaging section 200 may include an autofocus optical system, and the distance to the treatment tool or the blood vessel in the depth direction may be estimated from the lens position when the treatment tool or the blood vessel has been brought into focus through the autofocus process. Alternatively, the distance to the treatment tool or the blood vessel in the depth direction may be estimated from the brightness of the image by utilizing the fact that the brightness of illumination light applied from the end of the imaging section 200 decreases as the distance to the object increases.

According to the second embodiment, the distance information acquisition section 120 acquires the relative distance (three-dimensional distance) between the specific part and the treatment tool 210 as the distance information. The degree-of-relation acquisition section 130 increases the degree of relation as the relative distance decreases.

This makes it possible to notify the user that the degree of relation is high when the treatment tool 210 has approached the specific part. Therefore, it is possible to allow the user to determine whether or not the specific part is damaged (e.g., bleeding from a blood vessel occurs) when the procedure is further performed in a state in which the current positional relationship between the treatment tool 210 and the specific part is maintained. This makes it possible for the user to perform a procedure more safely.

According to a related-art technique (e.g., Patent Document 1), it is necessary to acquire tissue (safety-critical part) image data in advance using MRI or the like, and provide a position detection device that detects the position of a treatment tool. Therefore, the related-art technique has a problem in that a large-scale apparatus is required.

According to the second embodiment, the image acquisition section 110 acquires the captured image that has been captured by the imaging section 200 and includes an image of the specific part (e.g., blood vessel) and the treatment tool. The distance information acquisition section 120 detects the position of the treatment tool 210 and the position of the specific part from the captured image, and acquires the distance information about the distance from the treatment tool 210 to the specific part based on the detected position of the treatment tool 210 and the detected position of the specific part.

According to this configuration, since it is unnecessary to acquire image data of the specific part in advance, it is possible to detect a situation in which the treatment tool 210 is approaching the specific part in real time from the captured image, and notify the user of the situation as the degree of relation. Since it is unnecessary to provide a device that acquires image data of the specific part in advance, and a device (e.g., ultrasonic imaging device, MRI, or GPS transmitter/receiver) that detects the position of the treatment tool 210 without using the captured image, it is possible to implement a compact apparatus.

The position of the treatment tool and the position of the safety-critical part may be detected using a stereoscopic endoscope. In this case, however, the treatment tool and the safety-critical part are not necessarily captured. Therefore, it may be difficult to detect a situation in which the treatment tool is approaching (or is situated close to) the safety-critical part by merely utilizing image data captured using a stereoscopic endoscope. Patent Document 1 is silent about measures to solve this problem.

According to the second embodiment, it is possible to assign a detection mark to a part (e.g., the handle of the treatment tool) that is necessarily observed, and detect the position of the end of the treatment tool that is hidden behind tissue based on the position of the mark detected from the image and size information about the treatment tool (as described later). It is also possible to capture a blood vessel situated in a deep area of tissue using NBI (described later), and detect the position of the blood vessel. According to the second embodiment, it is possible to detect the position of the treatment tool and the position of the specific part from an image, and it is unnecessary to provide a large-scale apparatus.

### 4. Third embodiment

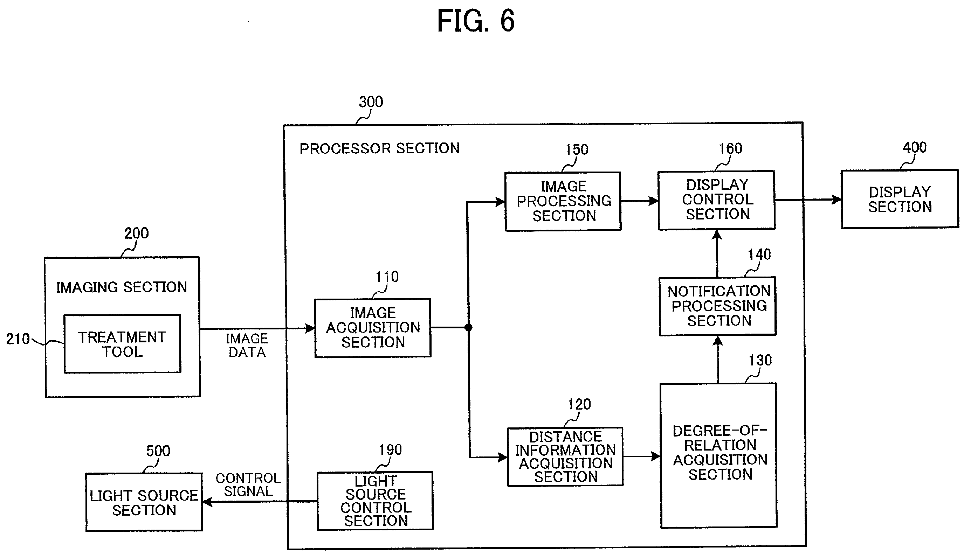

FIG. 6 illustrates a configuration example of an endoscope apparatus according to a third embodiment of the invention. The endoscope apparatus includes an imaging section 200, a processor section 300, a display section 400, and a light source section 500. The processor section 300 includes an image acquisition section 110, a distance information acquisition section 120, a degree-of-relation acquisition section 130, a notification processing section 140, an image processing section 150, a display control section 160, and a light source control section 190. Note that the same elements as those described above in connection with the first and second embodiments are indicated by the same reference signs (symbols), and description thereof is appropriately omitted.

In the third embodiment, an image captured using special light that makes it possible to capture a deep blood vessel is acquired. A deep blood vessel is detected from the captured image, and distance information about the distance from the treatment tool to the deep blood vessel is acquired using the depth of the deep blood vessel from the object. The degree of relation is acquired based on the distance information.

Specifically, the light source section 500 emits normal light (white light) that has a white wavelength band, and special light that has a specific wavelength band (as illumination light). For example, the light source section 500 includes a light source that emits the normal light, and a filter that allows the special light to pass through. The light source section 500 emits the normal light when the filter is not inserted into the optical path, and emits the special light when the filter is inserted into the optical path. Alternatively, the light source section 500 may include a light source that emits the normal light, and a light source that emits the special light, and emits the normal light or the special light by switching the light source.

The light source control section 190 controls the light source section 500, and causes the light source section 500 to selectively emit the normal light or the special light as illumination light. The normal light and the special light may be switched based on an instruction input by the user, or the normal light and the special light may be automatically emitted alternately, for example.

The specific wavelength band is a band that is narrower than the wavelength band (e.g., 380 to 650 nm) of white light (i.e., narrow-band imaging (NBI)), and is the wavelength band of light absorbed by hemoglobin in blood. More specifically, the wavelength band of light absorbed by hemoglobin is 390 to 445 nm (B2 component (first narrow-band light)) or 530 to 550 nm (G2 component (second narrow-band light)). A wavelength band of 390 to 445 nm or 530 to 550 nm is selected from the viewpoint of absorption by hemoglobin and the ability to reach a surface area or a deep area of tissue. Note that the wavelength band is not limited thereto. For example, the lower limit of the wavelength band may decrease by about 0 to 10%, and the upper limit of the wavelength band may increase by about 0 to 10%, depending on a variation factor (e.g., experimental results for absorption by hemoglobin and the ability to reach a surface area or a deep area of tissue).

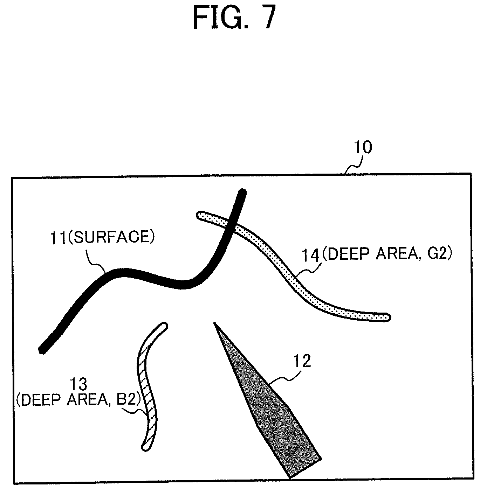

The imaging section 200 captures the special light using a normal RGB image sensor, for example. The image acquisition section 110 acquires the B component (i.e., B2 component) of the image input from the imaging section 200, and the G component (i.e., G2 component) of the image input from the imaging section 200, as a special light image. FIG. 7 illustrates an example of the special light image. Since a deep blood vessel that is present in an area deeper than the surface of tissue can be captured using the B2 component and the G2 component of the special light, the special light image includes a deep blood vessel 13 captured using the B2 component, and a deep blood vessel 14 captured using the G2 component. Since the B2 component and the G2 component differ in reachable depth from the surface of tissue, the deep blood vessel 13 and the deep blood vessel 14 differ in depth.

The distance information acquisition section 120 detects the blood vessel from the image captured using the B2 component and the image captured using the G2 component. Since the depth of a blood vessel that can be captured using each component is known in advance, the distance information acquisition section 120 acquires the depth of the blood vessel as the distance information about the blood vessel in the depth direction. The distance information acquisition section 120 calculates the three-dimensional distance from the treatment tool to the blood vessel based on the position of the treatment tool and the position of the blood vessel in the image plane, and the depth of the blood vessel captured using each component. The degree-of-relation acquisition section 130 converts the three-dimensional distance into the degree of relation in the same manner as described above in connection with the first embodiment.

In the third embodiment, the special light image may be acquired as described below. Specifically, the image acquisition section 110 may acquire the special light image by inputting the G component (i.e., G2 component) of the image input from the imaging section 200 to the R channel, and inputting the B component (i.e., B2 component) of the image input from the imaging section 200 to the G channel and the B channel. A lesion area (e.g., epidermoid cancer) that cannot be easily observed using the normal light can be displayed in brown or the like using such a special light image, and it is possible to prevent a situation in which the lesion area is missed.

Although an example in which the special light image is acquired while switching the normal light and the special light has been described above, the configuration is not limited thereto. For example, the normal light image and the special light image may be acquired using a frame sequential method. In this case, the light source section 500 sequentially emits R component light (normal light), G component light (normal light), B component light (normal light), B2 component light (special light), and G2 component light (special light). The imaging section 200 captures each component light using a monochrome image sensor, for example. The image acquisition section 110 acquires the normal light image from the R component image, the G component image, and the B component image, and acquires the special light image from the B2 component image and the G2 component image. Alternatively, the light source section 500 may emit only the normal light (white light), and the imaging section 200 may include an image sensor having a color filter that allows the special light to pass through, and capture the special light image using the image sensor.

According to the third embodiment, the image acquisition section 110 acquires the special light image as the captured image, the special light image including an image of the object that includes information within the specific wavelength band (B2 component and G2 component). The distance information acquisition section 120 acquires information about the depth (i.e., the depth of a deep blood vessel that can be captured using each of the B2 component and the G2 component) from the surface of the object to the specific part (e.g., deep blood vessels 13 and 14 (see FIG. 7)) captured using the special light image. The distance information acquisition section 120 acquires the distance information about the distance between the specific part and the treatment tool 210 based on the acquired information about the depth.

According to this configuration, it is possible to more accurately capture the specific part of the object including the specific part situated in a deep area by applying illumination light within the specific wavelength band. It is possible to acquire the position of the specific part as three-dimensional information by acquiring the information about the depth of the specific part situated in a deep area that has been captured using the special light. This makes it possible to accurately acquire the distance between the treatment tool 210 and the specific part (i.e., information that also includes the distance in the depth direction) as the degree of closeness, and notify the user of the acquired distance as more accurate information about the degree of relation.

### 5. Fourth embodiment

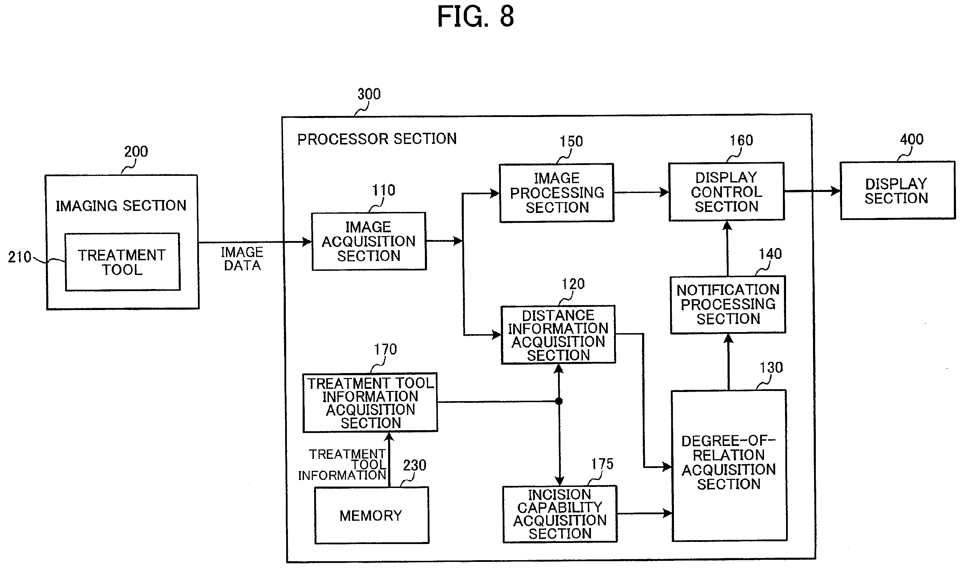

FIG. 8 illustrates a configuration example of an endoscope apparatus according to a fourth embodiment of the invention. The endoscope apparatus includes an imaging section 200, a processor section 300, and a display section 400. The processor section 300 includes an image acquisition section 110, a distance information acquisition section 120, a degree-of-relation acquisition section 130, a notification processing section 140, an image processing section 150, a display control section 160, a treatment tool information acquisition section 170, an incision capability acquisition section 175, and a memory 230. Note that the same elements as those described above in connection with the first to third embodiments are indicated by the same reference signs (symbols), and description thereof is appropriately omitted.

Although an example in which the treatment tool 210 is a knife is described below, the treatment tool 210 is not limited to a knife, and may be an arbitrary treatment tool that may damage a blood vessel.

In the fourth embodiment, the incision capability (degree of incision) of the knife is acquired based on the characteristic information about the knife (i.e., treatment tool 210), and the degree of relation is acquired based on the incision capability and the distance information from the knife to a blood vessel.

Specifically, the treatment tool information acquisition section 170 acquires the characteristic information about the knife (treatment tool 210) as the treatment tool information. The characteristic information about the knife is stored in the memory 230 included in the processor section 300, for example. The processor section 300 determines the treatment tool, and acquires the treatment tool information from the memory 230. Information about the application, the target part, the size, the shape, and the like of the knife is stored in the memory 230 as the characteristic information. The characteristic information about the knife may be input as the operation information about the user. For example, when the knife is an electrosurgical knife, the user sets the current that is caused to flow through the knife, the voltage that is applied to the knife, and the like. Alternatively, the user sets a knife procedure mode (e.g., an incision mode in which only an incision operation is performed, or a bleeding arrest mode in which a bleeding arrest operation is performed at the same time as an incision operation).

The incision capability acquisition section 175 acquires the incision capability of the knife based on the treatment tool information (i.e., the characteristic information about the knife) acquired by the treatment tool information acquisition section 170. For example, the incision capability acquisition section 175 stores a look-up table that links the treatment tool information with the incision capability, and acquires the incision capability by referring to the look-up table. Alternatively, the incision capability acquisition section 175 may acquire the incision capability using a function that calculates the incision capability using the performance of the treatment tool as an argument. The term "incision capability" used herein refers to information that represents the capability of the knife to incise tissue. For example, the term "incision capability" used herein refers to information that represents the range (e.g., length or depth) in which tissue is incised when a current is caused to flow through the knife (or when a voltage is applied to the knife) according to an instruction issued by the user.

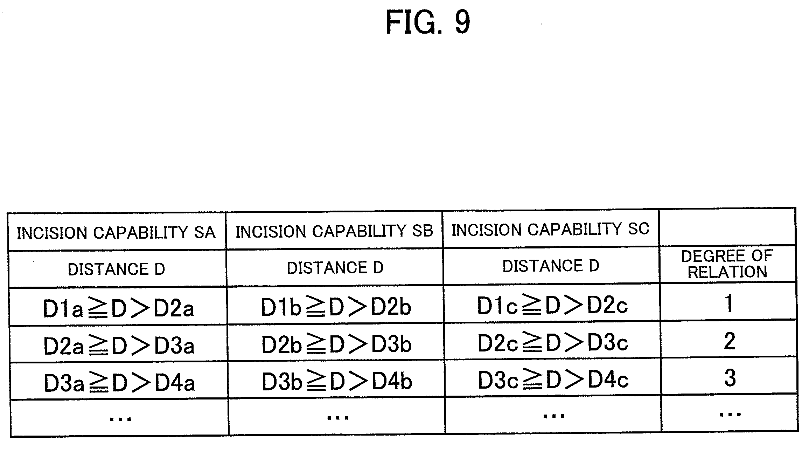

The degree-of-relation acquisition section 130 acquires the degree of relation based on the incision capability of the knife and the distance information about the distance from the knife to a blood vessel. For example, the degree-of-relation acquisition section 130 acquires the degree of relation by referring to the look-up table illustrated in FIG. 9 in which the relationship between the distance D and the degree of relation is defined with respect to incision capabilities SA to SC. In the example illustrated in FIG. 9, when the relationship "SA<SB<SC" is satisfied, an identical distance D satisfies D1a≥D>D2a, D2b≥D>D3b, and D3c≥D>D4c, for example. In this case, the degree of relation is 1 when the incision capability is SA, is 2 when the incision capability is SB, and is 3 when the incision capability is SC. Specifically, the degree of relation increases with respect to an identical distance D as the incision capability increases. Alternatively, the degree-of-relation acquisition section 130 may calculate the degree of relation using a function that links the incision capability and the distance information with the degree of relation. For example, when the incision capability is referred to as S, and the distance is referred to as D, the degree of relation is represented by f(S, D)=βS/D. Note that β is a given coefficient.

Note that the position of the tip of the knife may be estimated from the treatment tool information. In this case, the distance information acquisition section 120 acquires physical information (e.g., length and width) about the end (tip) of the treatment tool from the treatment tool information acquired by the treatment tool information acquisition section 170. For example, the tip of the knife may be hidden behind tissue when tissue is incised using the knife, and it may be impossible to determine the tip of the knife from the image. In order to deal with such a situation, a mark is provided to a part (e.g., the handle (grip) of the knife) of the treatment tool 210 other than the end (tip) thereof. The position of the mark is determined from the captured image, and the distance from the mark to the tip of the knife is acquired using the physical information about the knife. The position of the tip of the knife is thus determined, and the distance information about the distance from the tip of the knife to a blood vessel is acquired based the position of the tip of the knife. Although an example in which the knife procedure mode is the incision mode has been described above, the bleeding arrest capability may be acquired instead of the incision capability when the knife procedure mode is the bleeding arrest mode.

When the position of the tip of the knife has been estimated as described above, the notification processing section 140 may display the position of the tip of the knife on the display section 400. In this case, the user can also determine the degree of relation between the treatment tool 210 and a blood vessel. Specifically, it is possible to notify the user of the degree of relation by displaying the position of the tip of the knife. This makes it possible to notify the user of the position of the tip of the knife even when the tip of the knife is hidden behind tissue, and reduce the possibility that the user unintentionally damages a blood vessel.

According to the fourth embodiment, the endoscope apparatus includes the treatment tool information acquisition section 170 and the incision capability acquisition section 175. The treatment tool information acquisition section 170 acquires performance information (e.g., preset current value, preset voltage value, or physical information (e.g., size information)) about the treatment tool 210 (e.g., knife or electrosurgical knife) as the treatment tool information, the performance information relating to the incision capability of the treatment tool 210. The incision capability acquisition section 175 acquires information (e.g., incision range) about the incision capability of the treatment tool 210 based on the performance information about the treatment tool 210. The degree-of-relation acquisition section 130 increases the degree of relation as the incision capability of the treatment tool 210 increases (see FIG. 9, for example).

It is possible to acquire the degree of relation corresponding to the performance information about the treatment tool 210 by acquiring the performance information about the treatment tool 210. Specifically, it is possible to acquire the incision capability from the performance information, more accurately calculate the degree of relation taking account of the incision capability in addition to the distance information, and notify the user of the calculated degree of relation. For example, the incision capability of the treatment tool 210 changes corresponding to the preset current value and the like. According to the fourth embodiment, it is possible to notify the user of the degree of relation that corresponds to a change in incision capability.

The distance information acquisition section 120 detects the position of the mark provided to the treatment tool 210 from the captured image, and acquires the distance information based on the detected position of the mark. The mark is not particularly limited as long as the mark can be recognized within the captured image. For example, the mark may be a mark that is formed of a material that differs in reflectance from the treatment tool 210, a mark that is processed or stamped to have a shape or the like that can be recognized by image processing, a mark that is provided with a color by which the mark can be easily distinguished from tissue, or the like.

More specifically, the endoscope apparatus includes the treatment tool information acquisition section 170 that acquires the size information (physical information) about the treatment tool 210 as the treatment tool information. The distance information acquisition section 120 determines the position of the end of the treatment tool 210 from the position of the mark detected from the captured image and the size information about the treatment tool 210, and acquires the distance information about the distance from the determined position of the end of the treatment tool 210 to the specific part.

This makes it possible to detect the end of the treatment tool 210 as the three-dimensional position information even when the end of the treatment tool 210 cannot be determined within the captured image. Therefore, it is possible to accurately calculate the distance information about the distance from the end of the treatment tool 210 to the specific part, and notify the user of a more accurate degree of relation. Since the distance from the treatment tool 210 to the specific part can be accurately detected from the captured image without using a method other than image recognition, the configuration of the apparatus can be simplified.

### 6. Fifth embodiment

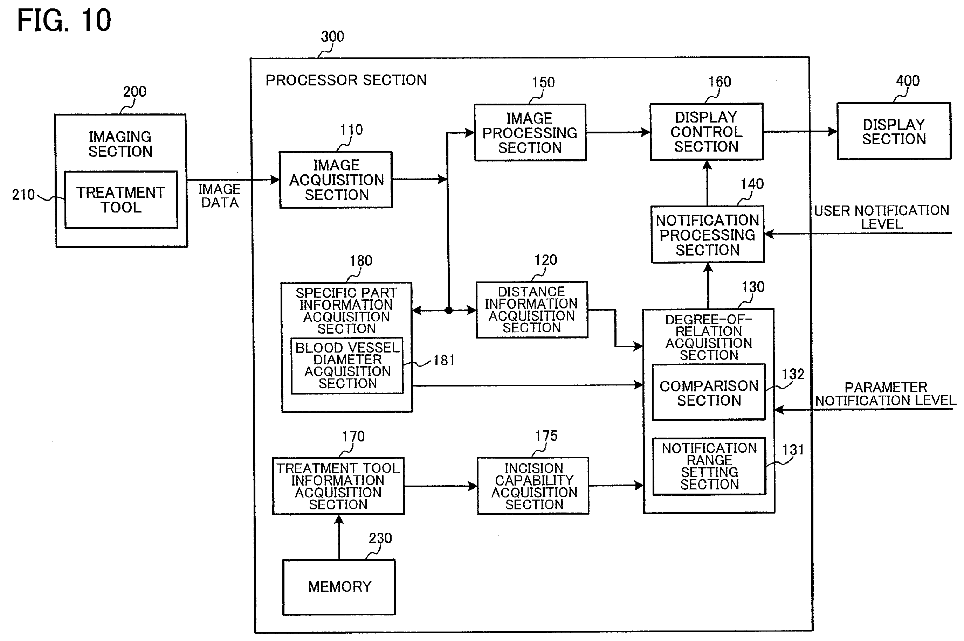

FIG. 10 illustrates a configuration example of an endoscope apparatus according to a fifth embodiment of the invention. The endoscope apparatus includes an imaging section 200, a processor section 300, and a display section 400. The processor section 300 includes an image acquisition section 110, a distance information acquisition section 120, a degree-of-relation acquisition section 130, a notification processing section 140, an image processing section 150, a display control section 160, a treatment tool information acquisition section 170, an incision capability acquisition section 175, a specific part information acquisition section 180, and a memory 230. Note that the same elements as those described above in connection with the first to fourth embodiments are indicated by the same reference signs (symbols), and description thereof is appropriately omitted.

In the fifth embodiment, the characteristic information about the specific part is acquired, and the degree of relation is acquired based on the characteristic information about the specific part and the distance information about the distance from the treatment tool 210 to the specific part. Although an example in which the specific part is a blood vessel, and the characteristic information about the blood vessel is the blood vessel diameter is described below, the configuration is not limited thereto. For example, the characteristic information may be the depth or the like of the blood vessel captured using the special light.

The specific part information acquisition section 180 includes a blood vessel diameter acquisition section 181. The specific part information acquisition section 180 detects a blood vessel from the captured image acquired by the image acquisition section 110, and the blood vessel diameter acquisition section 181 acquires the blood vessel diameter (width) as the characteristic information about the blood vessel based on the detection result. Since a blood vessel normally has a circular cross-sectional shape, the width of the blood vessel within the captured image is determined to be the blood vessel diameter. The information about the blood vessel diameter is linked to the position information about the blood vessel, and output to the degree-of-relation acquisition section 130. Note that the distance information acquisition section 120 may receive the position information about the blood vessel from the specific part information acquisition section 180, and acquire the distance information, or may detect a blood vessel from the captured image, and acquire the distance information.

The degree-of-relation acquisition section 130 acquires the degree of relation based on the blood vessel diameter and the distance information about the distance from the treatment tool 210 to the blood vessel. Specifically, the degree-of-relation acquisition section 130 acquires the degree of relation that increases with respect to an identical distance D as the blood vessel diameter increases. For example, the degree-of-relation acquisition section 130 stores a look-up table (e.g., the look-up table illustrated in FIG. 9 in which the incision capability is replaced by the blood vessel diameter) that links the distance and the degree of relation with the blood vessel diameter, and acquires the degree of relation by referring to the look-up table. Alternatively, the degree-of-relation acquisition section 130 may calculate the degree of relation using a function that links the blood vessel diameter and the distance information with the degree of relation. For example, when the blood vessel diameter is referred to as R, and the distance is referred to as D, the degree of relation is represented by f(R, D)=γR/D. Note that γ is a given coefficient.

The degree-of-relation acquisition section 130 determines whether or not to notify the user of the degree of relation based on a parameter notification level. Specifically, the degree-of-relation acquisition section 130 includes a notification range setting section 131 and a comparison section 132 (parameter comparison section).

The parameter notification level is input to the notification range setting section 131, the parameter notification level being set by the user, for example. The notification range setting section 131 sets the parameter range (hereinafter referred to as "notification range") based on the parameter notification level, the user being notified of the degree of relation when the parameter is included within the parameter range (notification range). When the parameter is the blood vessel diameter, the parameter notification level is a blood vessel diameter threshold value, and the user is notified of the degree of relation when the blood vessel diameter is larger than the threshold value, for example. Alternatively, a plurality of threshold values may be input as the parameter notification level. For example, a first threshold value and a second threshold value may be input as the parameter notification level, and the notification range may be determined to be a first notification range when the blood vessel diameter is larger than the first threshold value and smaller than the second threshold value, and determined to be a second notification range when the blood vessel diameter is larger than the second threshold value.

The comparison section 132 compares the blood vessel diameter with the notification range, and outputs a notification flag to the notification processing section 140 when the blood vessel diameter is within the notification range. The notification processing section 140 performs the notification process when the notification flag has been received. When a plurality of notification ranges (e.g., first notification range and second notification range (see above)) have been set, the notification method (e.g., the display color, the display size, or the blinking speed) may be changed corresponding to the notification range.

Note that a notification level that corresponds to characteristic information other than the blood vessel diameter may be input as the parameter notification level. For example, a notification level that corresponds to the blood vessel depth when captured using the special light may be input as the parameter notification level, or a notification level that corresponds to the distance information may be input as the parameter notification level, or a notification level that corresponds to the characteristic information (e.g., the incision capability of the knife) about the treatment tool 210 may be input as the parameter notification level. The comparison section 132 may output the notification flag when one parameter is within the notification range, or may output the notification flag when a plurality of parameters are within the notification range.

The notification processing section 140 receives a user notification level (that has been set by the user, for example), and determines whether or not to notify the user of the degree of relation based on the user notification level. The user notification level is a threshold value that is used to determine whether or not to notify the user of the degree of relation, and the notification processing section 140 notifies the user of the degree of relation when the degree of relation is higher than the threshold value. Alternatively, the user notification level includes a first threshold value and a second threshold value that are used to determine whether or not to notify the user of the degree of relation, and the notification processing section 140 notifies the user of the degree of relation when the degree of relation is larger than the first threshold value and smaller than the second threshold value. A notification level that differs corresponding to the user may be set as the user notification level.

According to the fifth embodiment, the endoscope apparatus includes the specific part information acquisition section 180 that acquires information about the blood vessel diameter as blood vessel information, the blood vessel diameter being the diameter of a blood vessel that is the specific part. The degree-of-relation acquisition section 130 increases the degree of relation as the blood vessel diameter increases.

Specifically, since the blood vessel diameter is acquired from the image as the specific part information, it is possible to variably control the degree of relation corresponding to the thickness of the blood vessel. The level of risk when the treatment tool 210 has damaged a blood vessel is normally high (e.g., a large amount of bleeding occurs, or it is difficult to arrest bleeding) when the thickness of the blood vessel is large. According to the fifth embodiment, it is possible to notify the user of the degree of relation that corresponds to the level of risk.

According to the fifth embodiment, the degree-of-relation acquisition section 130 includes the notification range setting section 131 that sets a blood vessel diameter notification range, the user being notified (of the degree of relation) when the blood vessel diameter is within the blood vessel diameter notification range, and the comparison section 132 that performs the comparison process on the blood vessel diameter and the blood vessel diameter notification range. The notification processing section 140 performs the notification process based on the degree of relation when the comparison section 132 has determined that the blood vessel diameter is within the blood vessel diameter notification range.

This makes it possible to control whether or not to notify the user of the degree of relation corresponding to whether or not the blood vessel diameter is within the notification range. For example, a blood vessel diameter threshold value may be input as the user notification level, and the blood vessel diameter notification range may be set to be a range in which the blood vessel diameter is larger than the threshold value. In this case, the user is notified of the degree of relation only when the blood vessel diameter is larger than the threshold value, and is not notified of the degree of relation when the blood vessel diameter is smaller than the threshold value. It is possible to notify the user of the degree of relation only when the user should be notified of the degree of relation (e.g., when the blood vessel diameter is large, and bleeding easily occurs) by notifying the user of the degree of relation when the desired condition is satisfied. This makes it possible suppress a situation in which the user is frequently (unnecessarily) notified of the degree of relation.

According to the fifth embodiment, the notification range setting section 131 sets an incision capability notification range, the user being notified (of the degree of relation) when the incision capability is within the incision capability notification range. The comparison section 132 performs the comparison process on the incision capability and the incision capability notification range. The notification processing section 140 performs the notification process based on the degree of relation when the comparison section 132 has determined that the incision capability is within the incision capability notification range.

This makes it possible to control whether or not to notify the user of the degree of relation corresponding to whether or not the incision capability is within the incision capability notification range. For example, it is possible to notify the user of the degree of relation only when the incision capability is higher than a threshold value. This makes it possible to notify the user of the degree of relation only when the user should be notified of the degree of relation (e.g., when the incision capability is high, and bleeding easily occurs), and suppress a situation in which the user is frequently (unnecessarily) notified of the degree of relation.

According to the fifth embodiment, the specific part information acquisition section 180 acquires the information about the blood vessel diameter as the specific part information, the blood vessel diameter being the diameter of a blood vessel that is the specific part. The notification processing section 140 performs the notification process based on the degree of relation when it has been determined that the incision capability is within the incision capability notification range, and the blood vessel diameter is larger than a threshold value (i.e., parameter notification level or user notification level (or may be a given threshold value)).

This makes it possible to control whether or not to notify the user of the degree of relation corresponding to the incision capability and the blood vessel diameter. For example, the risk of bleeding is high when the blood vessel diameter is large. However, the blood vessel diameter at which the risk of bleeding is high differs corresponding to the user's skill. It is possible to change the blood vessel diameter at which the user is notified that the treatment tool has approached the blood vessel by changing the threshold value (blood vessel diameter user notification level) corresponding to the user.

According to the fifth embodiment, the notification range setting section 131 sets a depth notification range, the user being notified (of the degree of relation) when the depth of the specific part (e.g., blood vessel) is within the depth notification range. The comparison section 132 performs the comparison process on the depth of the specific part and the depth notification range. The notification processing section 140 performs the notification process based on the degree of relation when the comparison section 132 has determined that the depth of the specific part is within the depth notification range.

This makes it possible to control whether or not to notify the user of the degree of relation corresponding to whether or not the depth of the specific part is within the notification range. For example, it is possible to notify the user of the degree of relation only when the depth of the specific part is smaller than a threshold value. This makes it possible to notify the user of the degree of relation only when the user should be notified of the degree of relation (e.g., when the depth of the specific part is small, and the treatment tool (having a specific incision capability) can reach the specific part), and suppress a situation in which the user is frequently (unnecessarily) notified of the degree of relation.

According to the fifth embodiment, the notification range setting section 131 sets a relative distance notification range, the user being notified (of the degree of relation) when the relative distance is within the relative distance notification range. The comparison section 132 performs the comparison process on the relative distance and the relative distance notification range. The notification processing section 140 performs the notification process based on the degree of relation when the comparison section 132 has determined that the relative distance is within the relative distance notification range.

This makes it possible to control whether or not to notify the user of the degree of relation corresponding to whether or not the distance from the treatment tool 210 to the specific part is within the notification range. For example, it is possible to notify the user of the degree of relation only when the distance is shorter than a threshold value. This makes it possible to notify the user of the degree of relation only when the user should be notified of the degree of relation (e.g., when the treatment tool 210 is situated close to the specific part, and the specific part may be cut by the treatment tool 210 (having a specific incision capability)), and suppress a situation in which the user is frequently (unnecessarily) notified of the degree of relation.

Note that whether or not to perform the notification process may be determined based on only one of the plurality of parameters (i.e., blood vessel diameter, incision capability, depth of specific part, and relative distance) (notification ranges), or may be determined based on a combination of some of the plurality of parameters (notification ranges).

According to the fifth embodiment, the notification processing section 140 sets a degree-of-relation notification range, the user being notified of the degree of relation when the degree of relation is within the degree-of-relation notification range, and performs the notification process based on the degree of relation when it has been determined that the degree of relation is within the degree-of-relation notification range.

This makes it possible to control whether or not to notify the user of the degree of relation corresponding to whether or not the degree of relation is within the notification range. For example, a threshold value that is compared with the degree of relation may be set as the user notification level corresponding to each user, and the user may be notified of the degree of relation when the degree of relation is higher than the threshold value. This makes it possible to customize the notification level corresponding to the user who desires to be notified of the degree of relation only when the degree of relation is high (e.g., when the treatment tool is situated close to the specific part, or the blood vessel diameter is large), and the user who desires to be notified of the degree of relation even when the degree of relation is low (e.g., when the treatment tool is not situated close to the specific part, or the blood vessel diameter is small).

### 7. Sixth embodiment

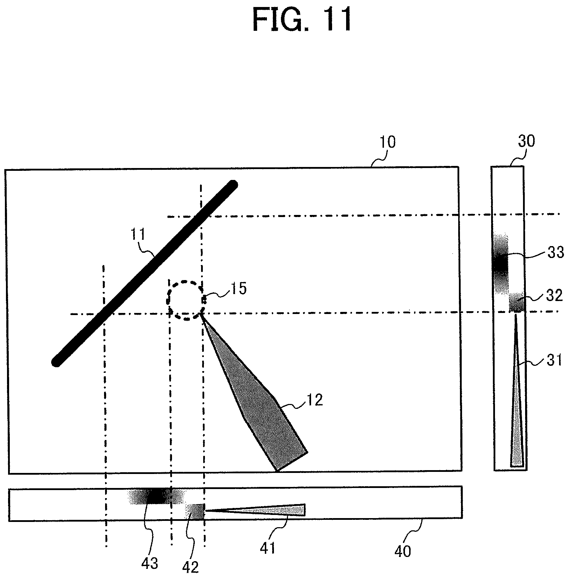

FIG. 11 illustrates an example of a degree-of-relation notification process according to a sixth embodiment of the invention. In the sixth embodiment, the incision range of the knife (treatment tool) is displayed on the display section 400 as the degree of relation.

Specifically, the position of the tip of the knife is detected from the captured image (or estimated from the mark and the physical information about the knife), and the incision capability of the knife is acquired from the characteristic information about the knife. The incision range is determined based on the incision capability, and displayed on the display section 400.

As illustrated in FIG. 11, an incision range 15 is displayed at the tip (detected or estimated tip position) of a knife 12 within a captured image display area 10, for example.

Alternatively, an incision range display area 30 in the vertical direction (i.e., the vertical direction of the screen) and an incision range display area 40 in the horizontal direction (i.e., the transverse direction of the screen) may be provided. An indicator 31 that represents the treatment tool 12, a bar 32 that represents the incision range, and a bar 33 that represents a blood vessel 11 are displayed within the display area 30. The bar 32 represents the incision range in the vertical direction using gradation (e.g., blue), and is displayed in a thick color at a position close to the end of the treatment tool 12 (i.e., a position at which the incision capability is high). The bar 33 represents the blood vessel 11 in the vertical direction using gradation (e.g., red), and is displayed in a thick color at a position close to the end of the treatment tool 12 (i.e., a position at which the possibility of incision is high). Likewise, an indicator 41 that represents the treatment tool 12 (in the horizontal direction), a bar 42 that represents the incision range, and a bar 43 that represents the blood vessel 11 are displayed within the display area 40.

Note that the degree of relation described above in connection with the first to fifth embodiments may be displayed in addition to the incision range. When the end of the treatment tool 12 cannot be observed within the image (e.g., when the end of the treatment tool 12 is hidden behind tissue), the position of the end of the treatment tool 12 may be estimated as described above in connection with the fourth embodiment, and displayed within the display area 10. The depth of the blood vessel acquired using the special light may be displayed in addition to the position of the blood vessel (see FIG. 11). The user can more accurately operate the treatment tool, and reduce the risk of bleeding even when the end of the treatment tool cannot be observed within the image by displaying the depth of the blood vessel.

According to the sixth embodiment, the notification processing section 140 performs the notification process that displays the incision range (e.g., 15, 32, and 42 in FIG. 11) on the display section 400, the incision range being an effective range of the incision capability of the treatment tool 210.

The notification processing section 140 performs the notification process that displays at least one of the depth information and the distance information (e.g., 33 and 43 in FIG. 11) about the specific part (e.g., blood vessel) on the display section 400.

This makes it possible to present more detailed information about the degree of relation to the user as compared with the case of displaying the degree of relation using a numerical value range (color) (see FIG. 4). In particular, it is possible to more accurately notify the user of the degree of relation when a plurality of specific parts are present in different directions when viewed from the treatment tool 210, or when a plurality of specific parts overlap each other in the depth direction. Therefore, the user can more appropriately determine the level of risk. Since the user can observe the degree of relation displayed within the monitor screen, the user can determine whether or not the settings (e.g., the settings relating to the incision capability (e.g., current value)) of the treatment tool 210 are appropriate by observing the screen. When the user notification level is set as described in connection with the fifth embodiment, it is possible to check the degree of relation within the monitor screen when setting the user notification level. This makes it possible to set an optimum user notification level.

### 8. Modifications

Note that the embodiments of the invention are not limited to the first to sixth embodiments. For example, the following configurations may also be employed.

For example, the notification processing section 140 may include a notification termination section that stops the notification process that notifies the user of the degree of relation when a given condition has been satisfied after the notification processing section 140 has performed the notification process based on the degree of relation output (transmitted) from the degree-of-relation acquisition section 130. For example, the notification process is automatically stopped when the distance (relative distance or two-dimensional distance) from the treatment tool to the blood vessel has reached zero (i.e., when the treatment tool and the blood vessel are situated at an equal distance) after the notification processing section 140 has notified the user of the degree of relation, or when the user has pressed a stop button after the notification processing section 140 has notified the user of the degree of relation, or when a given time has elapsed after the notification processing section 140 has started the notification process.

The processor section 300 may include a prediction-notification section that predicts a possibility of bleeding, and notifies the notification processing section 140 of a possibility of bleeding. For example, the prediction-notification section is a bleeding determination section, and the bleeding determination section determines that there is a possibility of bleeding when a knife (e.g., electrosurgical knife) or the like is set to an incision mode instead of a bleeding arrest mode, and the knife has approached a blood vessel having a blood vessel diameter that is equal to or larger than a given threshold value. The notification processing section 140 notifies the user that there is a possibility of bleeding (e.g., displays information that represents that there is a possibility of bleeding on the display section 400) when notified by the prediction-notification section that there is a possibility of bleeding. The notification processing section 140 may instruct the user to change the treatment tool, or may instruct the user to change the mode set to the treatment tool (e.g., set the treatment tool to the bleeding arrest mode) when notified by the prediction-notification section that there is a possibility of bleeding.

According to this configuration, the notification processing section 140 stops the notification process when a given time has elapsed after the notification processing section 140 has started the notification process based on the degree of relation, or when the degree of relation has exceeded a given threshold value after the notification processing section 140 has started the notification process based on the degree of relation.

This makes it possible to suppress a situation in which the user is frequently notified of the degree of relation, and is bothered by the notification. For example, when the user intentionally incises the specific part, it is unnecessary to notify the user of the degree of relation after the treatment tool 210 has reached the specific part (or after the distance between the treatment tool 210 and the specific part has become equal to or shorter than a given distance). According to the above configuration, the notification process can be automatically when such a situation has occurred.

The specific part may be a blood vessel. The notification processing section 140 may include a bleeding determination section (not illustrated in the drawings) that determines a possibility that bleeding occurs from the blood vessel due to the treatment tool based on at least one of the specific part information and the treatment tool information. The notification processing section 140 may perform a process that notifies that there is a possibility that bleeding occurs from the blood vessel when the bleeding determination section has determined that there is a possibility that bleeding occurs from the blood vessel.

According to this configuration, since it is possible to notify the user of a possibility of bleeding, the user can select an appropriate treatment tool 210 (or its settings) corresponding to the notification. For example, the user can set the treatment tool 210 to a mode (bleeding arrest mode) in which bleeding can be arrested while making an incision. The user can also prevent occurrence of bleeding by operating the treatment tool 210 while observing the monitor screen so that the treatment tool 210 is not situated too close to the blood vessel. Moreover, the user can promptly take appropriate measures (e.g., perform a bleeding arrest operation) when bleeding has occurred since the user has been notified that there is a possibility that bleeding occurs.

The specific part information acquisition section 180 may acquire information about the blood vessel diameter as the specific part information, the blood vessel diameter being the diameter of a blood vessel. The bleeding determination section may determine that there is a possibility that bleeding occurs from the blood vessel when the degree of relation is higher than a degree-of-relation threshold value (e.g., given threshold value), and the blood vessel diameter is larger than a blood vessel diameter threshold value (e.g., given threshold value).

More specifically, the notification processing section 140 may perform the notification process based on the degree of relation when the degree of relation is higher than a second degree-of-relation threshold value (i.e., parameter notification level or user notification level (see the fifth embodiment) (may be a given threshold value)), or when the blood vessel diameter is larger than a second blood vessel diameter threshold value (i.e., parameter notification level or user notification level (see the fifth embodiment) (may be a given threshold value)). In this case, the degree-of-relation threshold value used when determining a possibility that bleeding occurs from the blood vessel may be larger than the second degree-of-relation threshold value, and the blood vessel diameter threshold value used when determining a possibility that bleeding occurs from the blood vessel may be larger than the second blood vessel diameter threshold value.

This makes it possible to notify the user of a possibility of bleeding only when the blood vessel diameter is large (i.e., when the level of risk is high if bleeding occurs), and prevent a situation in which the user is unnecessarily notified of a possibility of bleeding. Since the threshold value used when determining a possibility of bleeding is larger than the threshold value used when determining whether or not to notify the user of the degree of relation, the notification process starts when the treatment tool has approached the blood vessel (i.e., when the degree of relation has increased), and it is possible to notify the user of a possibility of bleeding when the treatment tool has further approached the blood vessel.

The treatment tool information acquisition section 170 may acquire mode information set to the treatment tool as the treatment tool information. The bleeding determination section may determine that there is a possibility that bleeding occurs from the blood vessel when the degree of relation is higher than the degree-of-relation threshold value, and the treatment tool is set to the incision mode.

This makes it possible to notify the user of a possibility of bleeding only when the treatment tool is set to a mode in which the incision capability is high when the user utilizes a treatment tool that can be set to a plurality of modes that differ in incision capability. Therefore, it is possible to prevent a situation in which the user is unnecessarily notified of a possibility of bleeding. For example, an electrosurgical knife can be set to a bleeding arrest mode in which importance is given to bleeding arrest as compared with incision by applying a relatively small amount of high-frequency electric power, and an incision mode in which importance is given to incision as compared with bleeding arrest by applying a relatively large amount of high-frequency electric power. The mode is switched based on an instruction issued by the user, for example.

The notification processing section 140 may include a treatment tool determination section (not illustrated in the drawings) that determines whether or not the treatment tool 210 that is currently used is appropriate for the specific part based on the specific part information and the treatment tool information. The notification processing section 140 may perform a process that notifies that the treatment tool 210 that is currently used should be changed to another treatment tool 210 that is appropriate for the specific part when the treatment tool determination section has determined that the treatment tool is not appropriate.

This makes it possible to notify the user that there is a possibility that the user uses an inappropriate treatment tool 210, and prompt the user to exchange the treatment tool 210. For example, it is possible to prompt the user to use the treatment tool 210 having a lower incision capability, or change the mode to the bleeding arrest mode, when the user uses the treatment tool 210 having a high incision capability (or the treatment tool 210 that is set to the incision mode) near the blood vessel.

The specific part information acquisition section 180 may acquire the information about the blood vessel diameter as the specific part information, the blood vessel diameter being the diameter of a blood vessel that is the specific part. The treatment tool determination section may determine that the treatment tool is not appropriate when the degree of relation is higher than a degree-of-relation threshold value (e.g., given threshold value), and the blood vessel diameter is larger than a blood vessel diameter threshold value (e.g., given threshold value).

More specifically, the notification processing section 140 may perform the notification process based on the degree of relation when the degree of relation is higher than a second degree-of-relation threshold value (i.e., parameter notification level or user notification level (see the fifth embodiment) (may be a given threshold value)), or when the blood vessel diameter is larger than a second blood vessel diameter threshold value (i.e., parameter notification level or user notification level (see the fifth embodiment) (may be a given threshold value)). In this case, the degree-of-relation threshold value used when determining whether or not the treatment tool is appropriate may be larger than the second degree-of-relation threshold value, and the blood vessel diameter threshold value used when determining whether or not the treatment tool is appropriate may be larger than the second blood vessel diameter threshold value.

This makes it possible to prompt the user to exchange the treatment tool only when the level of risk is high if bleeding occurs (i.e., if the blood vessel is damaged) (e.g., when the blood vessel diameter is large), and prevent a situation in which the user is unnecessarily prompted to exchange the treatment tool. Since the threshold value used when determining whether or not the treatment tool is appropriate is larger than the threshold value used when determining whether or not to notify the user of the degree of relation, the notification process starts when the treatment tool has approached the specific part (i.e., when the degree of relation has increased), and it is possible to notify the user that the treatment tool is not appropriate when the treatment tool has further approached the specific part.

The treatment tool information acquisition section 170 may acquire mode information set to the treatment tool as the treatment tool information. The treatment tool determination section may determine that the treatment tool is appropriate when the degree of relation is higher than a degree-of-relation threshold value, and the treatment tool is set to the incision mode.

This makes it possible to notify the user that the treatment tool is not appropriate only when the treatment tool is set to a mode in which the incision capability is high when the user utilizes a treatment tool that can be set to a plurality of modes that differ in incision capability (e.g., an electrosurgical knife that can set to the bleeding arrest mode and the incision mode). Therefore, it is possible to prevent a situation in which the user is unnecessarily notified that the treatment tool is not appropriate.

The embodiments to which the invention is applied and the modifications thereof have been described above. Note that the invention is not limited to the above embodiments and the modifications thereof. Various modifications and variations may be made of the above embodiments and the modifications thereof without departing from the scope of the invention. A plurality of elements described in connection with the above embodiments and the modifications thereof may be appropriately combined to implement various configurations. For example, some elements may be omitted from the elements described in connection with the above embodiments and the modifications thereof. Some elements among the elements described in connection with the above embodiments and the modifications thereof may be appropriately combined. Specifically, various modifications and applications are possible without materially departing from the novel teachings and advantages of the invention. Any term cited with a different term having a broader meaning or the same meaning at least once in the specification and the drawings can be replaced by the different term in any place in the specification and the drawings.

### REFERENCE SIGNS LIST

10: image display area, 11: blood vessel, 12: treatment tool, 13, 14: deep blood vessel, 15: incision range, 20: degree-of-relation display area, 30, 40: incision range display area, 31, 41: indicator that represents treatment tool, 32, 42: bar that represents incision range, 33, 43: bar that represents blood vessel, 110: image acquisition section, 120: distance information acquisition section, 130: degree-of-relation acquisition section, 131: notification range setting section, 132: comparison section, 140: notification processing section, 150: image processing section, 160: display control section, 170: treatment tool information acquisition section, 175: incision capability acquisition section, 180: specific part information acquisition section, 181: blood vessel diameter acquisition section, 190: light source control section, 200: imaging section, 210: treatment tool, 220: stereo optical system, 230: memory, 300: processor section, 400: display section, 500: light source section, D: Distance, SA to SC: incision capability

## Claims

1. An endoscope apparatus comprising:
a distance information acquisition section that acquires distance information about a distance between a specific part and a treatment tool;
a degree-of-relation acquisition section that acquires a degree of relation between the specific part and the treatment tool based on at least one of specific part information and treatment tool information, and the distance information, the specific part information being characteristic information about the specific part, and the treatment tool information being characteristic information about the treatment tool; and
a notification processing section that performs a notification process based on the degree of relation.

2. The endoscope apparatus as defined in claim 1, further comprising:
a treatment tool information acquisition section that acquires performance information about the treatment tool as the treatment tool information, the performance information relating to an incision capability of the treatment tool; and
an incision capability acquisition section that acquires information about the incision capability of the treatment tool based on the performance information about the treatment tool,
the degree-of-relation acquisition section increasing the degree of relation as the incision capability of the treatment tool increases.

3. The endoscope apparatus as defined in claim 2,
the notification processing section performing the notification process that displays an incision range on a display section, the incision range being an effective range of the incision capability of the treatment tool.

4. The endoscope apparatus as defined in claim 2,
the degree-of-relation acquisition section including:
a notification range setting section that sets an incision capability notification range, a user being notified of the degree of relation when the incision capability is within the incision capability notification range; and
a comparison section that performs a comparison process on the incision capability and the incision capability notification range,
the notification processing section performing the notification process based on the degree of relation when the comparison section has determined that the incision capability is within the incision capability notification range.

5. The endoscope apparatus as defined in claim 4, further comprising:
a specific part information acquisition section that acquires information about a blood vessel diameter as the specific part information, the blood vessel diameter being a diameter of a blood vessel that is the specific part,
the notification processing section performing the notification process based on the degree of relation when it has been determined that the incision capability is within the incision capability notification range, and the blood vessel diameter is larger than a threshold value.

6. The endoscope apparatus as defined in claim 1,
the distance information acquisition section detecting a position of a mark provided to the treatment tool from a captured image, and acquiring the distance information based on the detected position of the mark.

7. The endoscope apparatus as defined in claim 6, further comprising:
a treatment tool information acquisition section that acquires size information about the treatment tool as the treatment tool information,
the distance information acquisition section determining a position of an end of the treatment tool based on the position of the mark detected from the captured image and the size information about the treatment tool, and acquiring the distance information about a distance from the determined position of the end to the specific part.

8. The endoscope apparatus as defined in claim 1, further comprising:
an image acquisition section that acquires a special light image as a captured image, the special light image including an image of an object that includes information within a specific wavelength band,
the distance information acquisition section acquiring information about a depth from a surface of the object to the specific part captured within the special light image, and acquiring the distance information about the distance between the specific part and the treatment tool based on the acquired information about the depth.

9. The endoscope apparatus as defined in claim 8,
the degree-of-relation acquisition section including:
a notification range setting section that sets a depth notification range, a user being notified when the depth of the specific part is within the depth notification range; and
a comparison section that performs a comparison process on the depth of the specific part and the depth notification range,
the notification processing section performing the notification process based on the degree of relation when the comparison section has determined that the depth of the specific part is within the depth notification range.

10. The endoscope apparatus as defined in claim 8,
the notification processing section performing the notification process that displays at least one of the depth of the specific part and the distance information on a display section.

11. The endoscope apparatus as defined in claim 1,
the distance information acquisition section acquiring a relative distance between the specific part and the treatment tool as the distance information, and
the degree-of-relation acquisition section increasing the degree of relation as the relative distance decreases.

12. The endoscope apparatus as defined in claim 11,
the degree-of-relation acquisition section including:
a notification range setting section that sets a relative distance notification range, a user being notified when the relative distance is within the relative distance notification range; and
a comparison section that performs a comparison process on the relative distance and the relative distance notification range,
the notification processing section performing the notification process based on the degree of relation when the comparison section has determined that the relative distance is within the relative distance notification range.

13. The endoscope apparatus as defined in claim 1, further comprising:
a blood vessel information acquisition section that acquires information about a blood vessel diameter as the specific part information, the blood vessel diameter being a diameter of a blood vessel that is the specific part,
the degree-of-relation acquisition section increasing the degree of relation as the blood vessel diameter increases.

14. The endoscope apparatus as defined in claim 13,
the degree-of-relation acquisition section including:
a notification range setting section that sets a blood vessel diameter notification range, a user being notified when the blood vessel diameter is within the blood vessel diameter notification range; and
a comparison section that performs a comparison process on the blood vessel diameter and the blood vessel diameter notification range,
the notification processing section performing the notification process based on the degree of relation when the comparison section has determined that the blood vessel diameter is within the blood vessel diameter notification range.

15. The endoscope apparatus as defined in claim 1,
the notification processing section setting a degree-of-relation notification range, a user being notified when the degree of relation is within the degree-of-relation notification range, and performing the notification process based on the degree of relation when it has been determined that the degree of relation is within the degree-of-relation notification range.

16. The endoscope apparatus as defined in claim 1,
the notification processing section stopping the notification process when a given time has elapsed after the notification processing section has started the notification process based on the degree of relation, or when the degree of relation has exceeded a given threshold value after the notification processing section has started the notification process based on the degree of relation.

17. The image processing device as defined in claim 1,
the specific part being a blood vessel,
the notification processing section including a bleeding determination section that determines a possibility that bleeding occurs from the blood vessel due to the treatment tool based on at least one of the specific part information and the treatment tool information, and
the notification processing section performing a process that notifies that there is a possibility that bleeding occurs from the blood vessel when the bleeding determination section has determined that there is a possibility that bleeding occurs from the blood vessel.

18. The endoscope apparatus as defined in claim 17, further comprising:
a specific part information acquisition section that acquires information about a blood vessel diameter of the blood vessel as the specific part information,
the bleeding determination section determining that there is a possibility that bleeding occurs from the blood vessel when the degree of relation is higher than a degree-of-relation threshold value, and the blood vessel diameter is larger than a blood vessel diameter threshold value.

19. The endoscope apparatus as defined in claim 18,
the notification processing section performing the notification process based on the degree of relation when the degree of relation is higher than a second degree-of-relation threshold value, or when the blood vessel diameter is larger than a second blood vessel diameter threshold value,
the degree-of-relation threshold value used when determining a possibility that bleeding occurs from the blood vessel being larger than the second degree-of-relation threshold value, and
the blood vessel diameter threshold value used when determining a possibility that bleeding occurs from the blood vessel being larger than the second blood vessel diameter threshold value.

20. The endoscope apparatus as defined in claim 17, further comprising:
a treatment tool information acquisition section that acquires mode information set to the treatment tool as the treatment tool information,
the bleeding determination section determining that there is a possibility that bleeding occurs from the blood vessel when the degree of relation is higher than a degree-of-relation threshold value, and the treatment tool is set to an incision mode.

21. The endoscope apparatus as defined in claim 1,
the notification processing section including a treatment tool determination section that determines whether or not the treatment tool that is currently used is appropriate for the specific part based on the specific part information and the treatment tool information, and
the notification processing section performing a process that notifies that the treatment tool should be changed to the treatment tool that is appropriate for the specific part when the treatment tool determination section has determined that the treatment tool is not appropriate.

22. The endoscope apparatus as defined in claim 21, further comprising:
a specific part information acquisition section that acquires information about a blood vessel diameter as the specific part information, the blood vessel diameter being a diameter of a blood vessel that is the specific part,
the treatment tool determination section determining that the treatment tool is not appropriate when the degree of relation is higher than a degree-of-relation threshold value, and the blood vessel diameter is larger than a blood vessel diameter threshold value.

23. The endoscope apparatus as defined in claim 22,
the notification processing section performing the notification process based on the degree of relation when the degree of relation is higher than a second degree-of-relation threshold value, or when the blood vessel diameter is larger than a second blood vessel diameter threshold value,
the degree-of-relation threshold value used when determining whether or not the treatment tool is appropriate being larger than the second degree-of-relation threshold value, and
the blood vessel diameter threshold value used when determining whether or not the treatment tool is appropriate being larger than the second blood vessel diameter threshold value.

24. The endoscope apparatus as defined in claim 21, further comprising:
a treatment tool information acquisition section that acquires mode information set to the treatment tool as the treatment tool information,
the treatment tool determination section determining that the treatment tool is appropriate when the degree of relation is higher than a degree-of-relation threshold value, and the treatment tool is set to an incision mode.

25. The endoscope apparatus as defined in claim 1, further comprising:
an image acquisition section that acquires a captured image that has been captured by an imaging section and includes an image of the specific part and the treatment tool,
the distance information acquisition section detecting a position of the treatment tool and a position of the specific part from the captured image, and acquiring the distance information about the distance from the treatment tool to the specific part based on the detected position of the treatment tool and the detected position of the specific part.

26. A method for operating an endoscope apparatus comprising:
acquiring distance information about a distance between a specific part and a treatment tool;
acquiring a degree of relation between the specific part and the treatment tool based on at least one of specific part information and treatment tool information, and the distance information, the specific part information being characteristic information about the specific part, and the treatment tool information being characteristic information about the treatment tool; and
performing a notification process based on the degree of relation.

## Amended claims

### Amended claims under Art. 19.1 PCT

1. (Amended) An endoscope apparatus comprising:
a distance information acquisition section that acquires distance information about a distance between a specific part and a treatment tool;
a degree-of-relation acquisition section that acquires a degree of relation between the specific part and the treatment tool based on at least one of specific part information and treatment tool information, and the distance information, the specific part information being characteristic information about the specific part, and the treatment tool information being characteristic information about the treatment tool;
a notification processing section that performs a notification process based on the degree of relation;
a treatment tool information acquisition section that acquires performance information about the treatment tool as the treatment tool information, the performance information relating to an incision capability of the treatment tool; and
an incision capability acquisition section that acquires information about the incision capability of the treatment tool based on the performance information about the treatment tool,
the degree-of-relation acquisition section increasing the degree of relation as the incision capability of the treatment tool increases.

2. (Cancelled)

3. (Amended) The endoscope apparatus as defined in claim 1,
the notification processing section performing the notification process that displays an incision range on a display section, the incision range being an effective range of the incision capability of the treatment tool.

4. (Amended) The endoscope apparatus as defined in claim 1,
the degree-of-relation acquisition section including:
a notification range setting section that sets an incision capability notification range, a user being notified of the degree of relation when the incision capability is within the incision capability notification range; and
a comparison section that performs a comparison process on the incision capability and the incision capability notification range,
the notification processing section performing the notification process based on the degree of relation when the comparison section has determined that the incision capability is within the incision capability notification range.

5. The endoscope apparatus as defined in claim 4, further comprising:
a specific part information acquisition section that acquires information about a blood vessel diameter as the specific part information, the blood vessel diameter being a diameter of a blood vessel that is the specific part,
the notification processing section performing the notification process based on the degree of relation when it has been determined that the incision capability is within the incision capability notification range, and the blood vessel diameter is larger than a threshold value.

6. The endoscope apparatus as defined in claim 1,
the distance information acquisition section detecting a position of a mark provided to the treatment tool from a captured image, and acquiring the distance information based on the detected position of the mark.

7. The endoscope apparatus as defined in claim 6, further comprising:
a treatment tool information acquisition section that acquires size information about the treatment tool as the treatment tool information,
the distance information acquisition section determining a position of an end of the treatment tool based on the position of the mark detected from the captured image and the size information about the treatment tool, and acquiring the distance information about a distance from the determined position of the end to the specific part.

8. The endoscope apparatus as defined in claim 1, further comprising:
an image acquisition section that acquires a special light image as a captured image, the special light image including an image of an object that includes information within a specific wavelength band,
the distance information acquisition section acquiring information about a depth from a surface of the object to the specific part captured within the special light image, and acquiring the distance information about the distance between the specific part and the treatment tool based on the acquired information about the depth.

9. The endoscope apparatus as defined in claim 8,
the degree-of-relation acquisition section including:
a notification range setting section that sets a depth notification range, a user being notified when the depth of the specific part is within the depth notification range; and
a comparison section that performs a comparison process on the depth of the specific part and the depth notification range,
the notification processing section performing the notification process based on the degree of relation when the comparison section has determined that the depth of the specific part is within the depth notification range.

10. The endoscope apparatus as defined in claim 8,
the notification processing section performing the notification process that displays at least one of the depth of the specific part and the distance information on a display section.

11. The endoscope apparatus as defined in claim 1,
the distance information acquisition section acquiring a relative distance between the specific part and the treatment tool as the distance information, and
the degree-of-relation acquisition section increasing the degree of relation as the relative distance decreases.

12. The endoscope apparatus as defined in claim 11,
the degree-of-relation acquisition section including:
a notification range setting section that sets a relative distance notification range, a user being notified when the relative distance is within the relative distance notification range; and
a comparison section that performs a comparison process on the relative distance and the relative distance notification range,
the notification processing section performing the notification process based on the degree of relation when the comparison section has determined that the relative distance is within the relative distance notification range.

13. The endoscope apparatus as defined in claim 1, further comprising:
a blood vessel information acquisition section that acquires information about a blood vessel diameter as the specific part information, the blood vessel diameter being a diameter of a blood vessel that is the specific part,
the degree-of-relation acquisition section increasing the degree of relation as the blood vessel diameter increases.

14. The endoscope apparatus as defined in claim 13,
the degree-of-relation acquisition section including:
a notification range setting section that sets a blood vessel diameter notification range, a user being notified when the blood vessel diameter is within the blood vessel diameter notification range; and
a comparison section that performs a comparison process on the blood vessel diameter and the blood vessel diameter notification range,
the notification processing section performing the notification process based on the degree of relation when the comparison section has determined that the blood vessel diameter is within the blood vessel diameter notification range.

15. The endoscope apparatus as defined in claim 1,
the notification processing section setting a degree-of-relation notification range, a user being notified when the degree of relation is within the degree-of-relation notification range, and performing the notification process based on the degree of relation when it has been determined that the degree of relation is within the degree-of-relation notification range.

16. The endoscope apparatus as defined in claim 1,
the notification processing section stopping the notification process when a given time has elapsed after the notification processing section has started the notification process based on the degree of relation, or when the degree of relation has exceeded a given threshold value after the notification processing section has started the notification process based on the degree of relation.

17. The image processing device as defined in claim 1,
the specific part being a blood vessel,
the notification processing section including a bleeding determination section that determines a possibility that bleeding occurs from the blood vessel due to the treatment tool based on at least one of the specific part information and the treatment tool information, and
the notification processing section performing a process that notifies that there is a possibility that bleeding occurs from the blood vessel when the bleeding determination section has determined that there is a possibility that bleeding occurs from the blood vessel.

18. The endoscope apparatus as defined in claim 17, further comprising:
a specific part information acquisition section that acquires information about a blood vessel diameter of the blood vessel as the specific part information,
the bleeding determination section determining that there is a possibility that bleeding occurs from the blood vessel when the degree of relation is higher than a degree-of-relation threshold value, and the blood vessel diameter is larger than a blood vessel diameter threshold value.

19. The endoscope apparatus as defined in claim 18,
the notification processing section performing the notification process based on the degree of relation when the degree of relation is higher than a second degree-of-relation threshold value, or when the blood vessel diameter is larger than a second blood vessel diameter threshold value,
the degree-of-relation threshold value used when determining a possibility that bleeding occurs from the blood vessel being larger than the second degree-of-relation threshold value, and
the blood vessel diameter threshold value used when determining a possibility that bleeding occurs from the blood vessel being larger than the second blood vessel diameter threshold value.

20. The endoscope apparatus as defined in claim 17, further comprising:
a treatment tool information acquisition section that acquires mode information set to the treatment tool as the treatment tool information,
the bleeding determination section determining that there is a possibility that bleeding occurs from the blood vessel when the degree of relation is higher than a degree-of-relation threshold value, and the treatment tool is set to an incision mode.

21. The endoscope apparatus as defined in claim 1,
the notification processing section including a treatment tool determination section that determines whether or not the treatment tool that is currently used is appropriate for the specific part based on the specific part information and the treatment tool information, and
the notification processing section performing a process that notifies that the treatment tool should be changed to the treatment tool that is appropriate for the specific part when the treatment tool determination section has determined that the treatment tool is not appropriate.

22. The endoscope apparatus as defined in claim 21, further comprising:
a specific part information acquisition section that acquires information about a blood vessel diameter as the specific part information, the blood vessel diameter being a diameter of a blood vessel that is the specific part,
the treatment tool determination section determining that the treatment tool is not appropriate when the degree of relation is higher than a degree-of-relation threshold value, and the blood vessel diameter is larger than a blood vessel diameter threshold value.

23. The endoscope apparatus as defined in claim 22,
the notification processing section performing the notification process based on the degree of relation when the degree of relation is higher than a second degree-of-relation threshold value, or when the blood vessel diameter is larger than a second blood vessel diameter threshold value,
the degree-of-relation threshold value used when determining whether or not the treatment tool is appropriate being larger than the second degree-of-relation threshold value, and
the blood vessel diameter threshold value used when determining whether or not the treatment tool is appropriate being larger than the second blood vessel diameter threshold value.

24. The endoscope apparatus as defined in claim 21, further comprising:
a treatment tool information acquisition section that acquires mode information set to the treatment tool as the treatment tool information,
the treatment tool determination section determining that the treatment tool is appropriate when the degree of relation is higher than a degree-of-relation threshold value, and the treatment tool is set to an incision mode.

25. The endoscope apparatus as defined in claim 1, further comprising:
an image acquisition section that acquires a captured image that has been captured by an imaging section and includes an image of the specific part and the treatment tool,
the distance information acquisition section detecting a position of the treatment tool and a position of the specific part from the captured image, and acquiring the distance information about the distance from the treatment tool to the specific part based on the detected position of the treatment tool and the detected position of the specific part.

26. (Amended) A method for operating an endoscope apparatus comprising:
acquiring distance information about a distance between a specific part and a treatment tool;
acquiring a degree of relation between the specific part and the treatment tool based on at least one of specific part information and treatment tool information, and the distance information, the specific part information being characteristic information about the specific part, and the treatment tool information being characteristic information about the treatment tool;
acquiring performance information about the treatment tool as the treatment tool information, the performance information relating to an incision capability of the treatment tool;
acquiring information about the incision capability of the treatment tool based on the performance information about the treatment tool;
increasing the degree of relation as the incision capability of the treatment tool increases; and
performing a notification process based on the degree of relation.
